# EUROPÄISCHE PATENTSCHRIFT

(11) **EP 1 699 451 B1**
(45) Veröffentlichungstag und Bekanntmachung des Hinweises auf die Patenterteilung: **03.10.2007**
(21) Anmeldenummer: 04804278.2
(22) Anmeldetag: 23.12.2004
(51) Int. Cl.: A61K 31/366, A61K 31/20, A61K 31/137, A61K 45/06, A61P 11/08

(54) **VERWENDUNG VON SILIBININ MIT ALPHA-LIPONSÄURE ZUR BEHANDLUNG CHRONISCH OBSTRUKTIVER LUNGENERKRANKUNGEN**
USE OF SILIBININ TOGETHER WITH ALPHA-LIPONIC ACID FOR THE TREATMENT OF CHRONIC OBSTRUCTIVE PULMONARY DISEASES
UTILISATION DE SILIBININE AVEC UN ACIDE ALPHA-LIPOIQUE POUR LE TRAITEMENT DES BRONCHO-PNEUMOPATHIES CHRONIQUES OBSTRUCTIVES

(30) Priorität: 23.12.2003 DE 10360954
(43) Veröffentlichungstag der Anmeldung: 13.09.2006
(73) Patentinhaber: Esparma GmbH, 39171 Osterweddingen (DE); Imtm GmbH, 39104 Magedeburg (DE)
(72) Erfinder: ANSORGE, Siegfried, 39291 Hohenwarthe (DE); KOEGST, Dieter, 39175 Wahlitz (DE); TÄGER, Michael, 39326 Heinrichsberg (DE); FRIES, Gerhard, 39175 Wahlitz (DE)
(74) Vertreter: Pfenning, Meinig & Partner GbR
(86) Internationale Anmeldenummer: PCT/EP2004/014687
(87) Internationale Veröffentlichungsnummer: WO 2005/063234

(56) Entgegenhaltungen:
- EP-A- 1 442 743
- WO-A-01/68069
- WO-A-99/55326
- WO-A-02/096398
- WO-A-02/096414
- US-A- 5 972 993
- US-A1- 2002 002 136
- BUHL R ET AL: "ANTIOXIDANTS IN THE TREATMENT OF LUNG DISEASES ANTIOXIDANZIEN ZUR THERAPIE VON LUNGENERKRANKUNGEN" MEDIZINISCHE MONATSSCHRIFT FUER PHARMAZEUTEN, STUTTGART, DE, Bd. 19, Nr. 10, 1996, Seiten 287-294, XP009044433 ISSN: 0342-9601
- BERKSON B M: "A CONSERVATIVE TRIPLE ANTIOXIDANT APPROACH TO THE TREATMENT OF HEPATITIS C COMBINATION OF ALPHA LIPOIC ACID (THIOCTIC ACID), SILYMARIN, AND SELENIUM: THREE CASE HISTORIES" MEDIZINISCHE KLINIK, Bd. 3, 15. Oktober 1999 (1999-10-15), Seiten 84-89, XP000965112

## Beschreibung

Die Erfindung betrifft die Verwendung von mindestens einem Effektor des Glutathionmetabolismus nämlich Silibimin, zusammen mit α-Liponsäure und/oder deren Salze zur gleichzeitigen, getrennten oder zeitlich abgestuften zytoprotektiven Behandlung chronisch obstruktiver Lungenerkrankungen.

Chronisch obstruktive Lungenerkrankungen (chronische Bronchitis, chronic obstructive pulmonary diseases, COPD) zählen zu den zahlenmäßig am stärksten wachsenden Gesundheitsproblemen der modernen Industrienationen. Die Ursachen für diese Zunahme sind vielfältig, wobei Umweltfaktoren sowie ungünstige Lebensgewohnheiten einschließlich Nikotinabusus eine besondere Rolle spielen. Der jährlich durch direkte und indirekte Krankheitskosten entstehende volkswirtschaftliche Schaden stellt eine erhebliche Belastung dar und begründet vielfältige Maßnahmen für therapeutische und präventive Interventionen.

Einmal manifest gewordene chronische Lungenobstruktionen sind in der Regel nicht ursächlich therapierbar. Die Behandlung muss sich an der möglichst weitgehenden Reduktion der Symptomatik orientieren. Hierzu zählen Sekretolyse sowie Bronchodilatation. Mit Ausnahme leichterer Formen ist eine begleitende entzündungshemmende Therapie mit Cortikosteroiden zwingend erforderlich.

In den vergangenen Jahren wurde eine Reihe von experimentellen Arbeiten durchgeführt, in denen versucht wurde, pathophysiologische Zusammenhänge hinsichtlich der Lungenfunktionseinschränkung und zellulären Effektormechanismen sowohl der Lungenzelltypen als auch einwandernder bzw. residenter Immunzellen nachzuvollziehen.

Im Ergebnis zeichnete sich insbesondere neben gewebedestruierenden Prozessen, welche von matrixgebundenen und löslichen Proteasen vermittelt werden und letztlich zur Entstehung des Lungenemphysems führen, eine Dysregulation der Funktion lungentypischer Immunzellen ab. Im Vordergrund stehen hier Alveolarmakrophagen, die den zahlenmäßig größten Anteil mit über 83% der Immunzellen im bronchoalveolären Raum ausmachen. Der Vergleich von Alveolarmakrophagen Gesunder mit COPD Patienten zeigt eine deutlich verringerte Funktionalität der Alveolarmakrophagen in den Patientengruppen, welche hauptsächlich durch den Verlust der originären Phagozytosefähigkeit sowie der Bakterizidie gekennzeichnet ist und regelmäßig von einer Störung in der Homöostase der Zytokinproduktion begleitet wird.

Darüberhinaus konnte nachgewiesen werden, dass Alveolarmakrophagen von COPD Patienten und insbesondere von rauchenden COPD Patienten einen hochgradig geschädigten Thiol-Disulfidstatus aufweisen. Im Zusammenhang mit den Kenntnissen hinsichtlich einer direkten Korrelation von Thioldefekt und Funktionsstörung in anderen zellulären Systemen war der Schluss naheliegend, dass dieses pulmonale Thioldefizit eine pathophysiologische Schlüsselrolle bei der Entstehung und insbesondere der Unterhaltung der Erkrankung darstellt.

Die Feinregulation des Thiol-Disulfidstatus stellt eine der wichtigsten Grundvoraussetzungen biologischer Stoffwechselleistungen dar. Das zentrale Regulationselement innerhalb dieses Systems ist das Tripeptid Glutathion, welches intrazelluläre in reduzierter Form relativ hohe Konzentrationen (bis zu 10 mM) erreicht.

Neben dem Glutathion sind SH (Thiol)-Gruppen tragende Proteine intrazelluläre und insbesondere in zellmembrangebundener Form weitere bedeutende Bausteine des Thiol-Disulfidstatus jeder Zelle.

Der durch verschiedene Enzymklassen regulierte Metabolismus der Disulfidspaltung und Thiolgruppenbildung ist durch die Vielfalt seiner biologischen Funktionen u.a. bei zellulären Wachstums- und Differenzierungsprozessen einschließlich des programmierten Zelltodes sowie Zellschutz- und Entgiftungsmechanismen in seiner Intaktheit unabdingbar für jede normale Zellfunktion. Störungen in diesem System und Veränderungen der Konzentration der Thiole führen zu schwerwiegenden zellulären Funktionsstörungen, die nur im Einzelfall lokal begrenzt bleiben, in der Regel jedoch den gesamten Organismus beeinträchtigen.

So ist aus der DE 101 25 883 bekannt, dass insbesondere unter den Bedingungen einer hochgradig eingeschränkten Nierenfunktion und dadurch erforderlicher Nierenersatztherapie in Form der Hämo- bzw. Peritonealdialyse der zelluläre Thiol-Disulfidstoffwechsel schwer gestört ist. Diese Störung hat u.a. einen weitgehenden Verlust normaler Zellfunktionen, die der Phagozytosefähigkeit von Peritonealmakrophagen oder der Aktivierbarkeit von Lymphozyten, zur Folge.

Die DE 101 25 832 beschreibt Studien im Rahmen von Diabetes mellitus, bei denen eine Verschiebung des Redoxzustands zu Lasten reduzierten Glutathions als auch eine absolute Verringerung des Gesamtpools an Glutathion nachgewiesen werden konnte. Diese Störung kann durch eine Kombination von α-Liponsäure und Prothiolen behoben werden.

Ausgehend hiervon war es Aufgabe der vorliegenden Erfindung, ein Arzneimittel bereitzustellen, mit dem die Funktionalität der Alveolarmakrophagen bei chronisch obstruktiven Lungenerkrankungen wiederhergestellt werden kann und die damit in Verbindung stehende Störung in der Homöostase der Zytokinproduktion behoben werden kann.

Diese Aufgabe wird durch die Verwendung mit den Merkmalen des Anspruchs 1 gelöst. Die weiteren abhängigen Ansprüche zeigen vorteilhafte Weiterbildungen auf.

Erfindungsgemäß wird die Verwendung von Silibinin und/oder dessen Salze zusammen mit α-Liponsäure und/oder deren Salze zur gleichzeitigen, getrennten oder zeitlich abgestuften zytoprotektiven Behandlung chronisch obstruktiver Lungenerkrankungen gelehrt.

Es konnte gezeigt werden, dass durch die Applikation der erfindungsgemäß zur Anwendung kommenden Kombination von α-Liponsäure und Silibinin und/oder dessen Salze eine Normalisierung des primär verringerten Thiolstatus von Alveolarmakrophagen einsetzte. Die thiolstabilisierende Wirkung der Kombination überstieg dabei die der alleinigen Verwendung von α-Liponsäure oder der jeweiligen Effektoren nicht nur regelmäßig, vielmehr konnten auch superadditive Effekte nachgewiesen werden.

Die Restitution des Thiolstatus erfasste dabei sowohl intrazelluläre Thiole als auch membrangebundene SH-Gruppen und ist somit Ausdruck einer komplexen biologischen Regulation. Dieses Phänomen beruht darauf, dass Silibinin und/oder dessen Salze als Effektoren des Glutathionstoffwechsels einerseits intermediär entstehende freie Radikale eliminieren, und andererseits die Verfügbarkeit reduzierender Äquivalente für die Umwandlung der α-Liponsäure aus disulfidischer in reduzierte Form erhöhen und somit die Synthese induzierende Wirkung der α-Liponsäure auf den Thiol-Disulfidstatus verbessern. Die Restitution des Thiolstatus der Immunzellen wurde begleitet von einer Normalisierung der Phagozytoseaktivität als Ausdruck einer Regulation zentraler funktioneller Parameter.

Die erfindungsgemäß zur Anwendung kommenden Kombinationen von α-Liponsäure mit Silibinin und/oder dessen Salze können in den üblichen pharmakologischen Darreichungsformen oder als Instillat sowohl prophylaktisch als auch therapeutisch verabreicht werden. Die effektive Dosis ist dabei fallbezogen zu ermitteln und liegt bevorzugt im Bereich zwischen 30 und 1800 mg/d und besonders bevorzugt zwischen 200 und 600 mg/d α-Liponsäure.

In einer weiteren bevorzugten Variante wird als Effektor des Glutathionmetabolismus Silibinin eingesetzt. Die Dosis von Silibinin und/oder dessen Salze für die humanmedizinische Applikation beträgt beim Patienten dabei bevorzugt zwischen 20 und 1600 mg/d und besonders bevorzugt zwischen 300 und 800 mg/d.

Bei Silibinin handelt es sich um einen Vertreter natürlich vorkommender Polyhydroxyphenylchromanonverbindungen, welche als Flavolignane bekannt sind. Innerhalb der Gruppe der Flavolignane Silymarin als Extrakt der Mariendistelfrüchte beschrieben. Silymarin wiederum ist ein Komplex aus den Flavonoiden Silibinin, Silichristin, Silidianin sowie Bitterstoffen. Wie in DE 35 37 656 beschrieben, kann Silibinin einschließlich der Stellungsisomere aus diesem Komplex isoliert werden.

Die Verabreichung des beschriebenen Arzneimittels kann inhalativ, oral oder parenteral erfolgen. Das Arzneimittel kann dabei die Form eines Aerosols, z.B. als Staub-, Nebel-, Spray oder Inhalationsaerosol, einer Lösung, eines Granulats, eines Pulvers, einer Emulsion, einer Tablette und/oder einer Filmtablette besitzen.

Vorzugsweise kann das Arzneimittel weitere Additive, ausgewählt aus der Gruppe wässriger Lösungsmittel, Stabilisatoren, Suspensions-, Dispersions- und Benetzungsmittel enthalten.

Silibinin und/oder dessen Salze und die α-Liponsäure können dabei sowohl in einer einzigen Formulierung als auch in getrennten Formulierungen vorliegen.

Anhand der nachfolgenden Beispiele soll die erfindungsgemäße Verwendung näher erläutert werden.

### Vergleichsbeispiel 1

### Einfluss der Kombination von α-Liponsäure mit Ambroxol auf den zellulären Thiolstatus von Alveolarmakrophagen

Zur Verwendung kam die etablierte normale Alveolarmakrophagen-Zelllinie CRL 21-92 (NR8383 [AgC11x3A; NR8383.1]). Die Zellen wurden in speziellen Zellkulturmedien aufgenommen und in einem Begasungsbrutschrank bei 37°C, einer relativen Luftfeuchte von 98% und 5% relativem Luft-CO₂-Gehalt inkubiert. Um den Einfluss der erfindungsgemäß zur Anwendung kommenden Kombinationen auf den Thiolstatus thioldefizienter Alveolarmakrophagen zu prüfen, wurden diese artifiziell thioldepletiert. Dies erfolgte durch Kultivierung in thioldefizienten Medien (TDM) nach erprobten Verfahren [Free Radic Biol Med 2000; 29:1160-1165]. Vergleichskulturen unter Verwendung von Vollmedien (RPMI 1640) dienten der Definition des unter Kulturbedingungen bestmöglichen Normalwertes.

Die Bestimmung des interzellulären Thiolgehaltes auf Einzelzellebene erfolgte unter Verwendung von 5-Chloromethylfluoresceindiacetat (CMFDA) in der Durchflußzytofluorimetrie [Cytometry 1994; 15:349-358, Cytometry 1997; 29:76-82].

Primär nicht fluorogenes CMFDA wird dabei passiv von der Zelle aufgenommen. Über den Chlormethylrest erfolgt eine Bindung an zytoplasmatische Thiolgruppen.

Nach Abspaltung der Acetatreste durch unspezifische zelluläre Esterasen wird dieser, nun zellmembranimpermeabele Komplex bei einer Exitationswellenlänge λₑₓ = 490 nm mit einer Emissionswellenlänge λₑₘ = 520 nm fluorogen. Die mittlere Fluoreszenzintensität der Probe (10.000 Zellen) ist der Konzentration der intrazellulären Thiolgruppen direkt proportional.

Die Expression membrangebundener Thiolgruppen wurde ebenfalls durchflußzytofluorimetrisch ermittelt. Hierbei wurde Chloromethyltetramethylrhodamin (CMTMR) unter den Bedingungen eines blockierten Membranpotentials sowie einer gehemmten Diffusionskapazität der Zellen als Thiolkonjugat eingesetzt [Exp Hematol 1997; 25(7): 601-607]. Die Fluoreszenzintensität der gebunden Fluorochrommoleküle an der Zellmembran ist dabei wiederum proportional der Menge der Thiolgruppen an der Zelloberfläche. Immortalisierte Alveolarmakrophagen wurden in der oben beschriebenen Versuchsanordnung artifiziell thioldepletiert. Der Einfluss der erfindungsgemäß zur Anwendung kommenden Substanzen wurde über einen Zeitraum von 96 Stunden mittels Messung des intrazellulären Thiolgehaltes sowie der Membranexpression von Thiolen überprüft.

Es zeigte sich, dass beginnend nach 24 Stunden die Kombination von α-Liponsäure und Ambroxol in einem breiten Konzentrationsbereich von 100 nM bis 10 µM eine vollständige Restitution des Thiolstatus der Alveolarmakrophagen initiierte. Wie in Tabelle 1 dargestellt, wurden signifikante Steigerungen des Thiolgehaltes über den gesamten Untersuchungszeitraum für diese Ambroxoldosen nachgewiesen. Die alleinige Wirkung der α-Liponsäure wurde regelmäßig signifikant überschritten.

Tabelle 1 zeigt den Einfluss von α-Liponsäure in Kombination mit Ambroxol auf die intrazelluläre Thiolkonzentration einer Alveolarmakrophagen-Zelllinie [*: p<0,05, ANOVA, n=12].

**Tabelle 1:**

| Thiole, intrazellulär | 24h | 48h | 72h | 96h |
|---|---|---|---|---|
| (CMFDA)[%] | MW ± SD | MW ± SD | MW ± SD | MW ± SD |
| KO, normal RPMI1640 | 100,0 ± 38,0 | 100,0 ± 67,9 | 100,0 ± 24,2 | 100,0 ± 5,4 |
| KO, thioldefizient TDM | 65,0 ± 6,0 | 35,7 ± 13,3 | 59,6 ± 16,8 | 73,3 ± 12,4 |
| αLS[10,0 µg/ml] TDM | 70,2 ± 8,4 | 58,9 ± 10,4 | 78,1 ± 30,0 | 72,5 ± 19,1 |
| αLS [10,0 µg/ml) Ambroxol [0,1 µM] TDM | 93,6 ± 17,8* | 96,9 ± 63,6* | 117,8 ± 12,5* | 102,7 ± 27,7* |
| αLS [10,0 µg/ml) Ambroxol [1,0 µM] TDM | 91,7 ± 8,6* | 77,5 ± 32,0* | 101,5 ± 26,8* | 101,1 ± 8,3* |
| αLS[10,0 µg/ml) Ambroxol [10,0 µM] TDM | 80,6 ± 9,7* | 71,8 ± 32,0* | 115,6 ± 25,8* | 101,0 ± 7,1* |
| αLS [10,0 µg/ml] Ambroxol (100,0 µM] TDM | 70,3 ± 6,7* | 74,6 ± 30,1* | 101,4 ± 23,1* | 62,4 ± 15,9* |
| αLS [10,0 µg/ml) Ambroxol [1000,0 µM) TDM | 10,5 ± 7,3 | 40,7 ± 24,5 | 22,5 ± 5,2 | 20,5 ± 21,5 |

Tabelle 2 zeigt den Einfluss von α-Liponsäure in Kombination mit Ambroxol auf die membranständige Thiolkonzentration einer Alveolarmakrophagen-Zelllinie [*: p<0,05, ANOVA, n=12]. Tabelle 2 unterlegt die parallele Induktion von membranständigen Thiolen.

**Tabelle 2:**

| Thiole, membranständig | 24h | 48h | 72h | 96h |
|---|---|---|---|---|
| (CMTMR)[%] | MW ± SD | MW ± SD | MW ± SD. | MW ± SD |
| KO, normal RPMI1640 | 100,0 ± 52,6 | 100,0 ± 7,9 | 100,0 ± 44.9 | 100,0 ± 44,2 |
| KO, thioldefizient TDM | 75,9 ± 38, 2 | 86,1 ± 15,0 | 68,2 ± 3,3 | 82,7 ± 44,1 |
| αLS [10,0 µg/ml] TDM | 91,1 ± 66,3 | 165,5 ± 36,3 | 82,3 ± 42,1 | 118,9 ± 76,6 |
| αLS(10,0 µg/ml] Ambroxol [0,1 µM] TDM | 90,7 ± 55,7 | 176,3 ± 6,1* | 82,5 ± 31,6 | 122,6 ± 76,8* |
| αLS[10,0 µg/ml] Ambroxol [1,0 µM] TDM | 96,2 ± 55,4 | 161,1 ± 25,3* | 82,2 ± 23.9* | 144,9 ± 110.4* |
| αLS[10,0 µg/ml] Ambroxol [10,0 µM] TDM | 97,3 ± 51,7 | 165,8 ± 37,2* | 87,2 ± 31,0* | 125,8 ± 73.4* |
| αLS[10,0 µg/ml] Ambroxol [100,0 µM] TDM | 106,6 ± 61.2* | 170,3 ± 36,8* | 93,5 ± 28,9* | 117.0 ± 51.0* |
| αLS[10,0 µg/ml] Ambroxol [1000,0 µM] TDM | 110,7 ± 25,6 | 159,9 ± 18,2* | 95,1 ± 27,5 | 125,0 ± 15,7* |

### Beispiel 2

### Einfluss von α-Liponsäure in Kombination mit Silibinin auf den zellulären Thiolstatus von Alveolarmakrophagen

Immortalisierte Alveolarmakrophagen wurden in der im Beispiel 1 beschriebenen Versuchsanordnung artifiziell thioldepletiert. Der Einfluss der erfindungsgemäß zur Anwendung kommenden Substanzen wurde über einen Zeitraum von 96 Stunden mittels Messung des intrazellulären Thiolgehaltes sowie der Membranexpression von Thiolen überprüft.

Es zeigte sich, dass beginnend nach 24 Stunden die Kombination von α-Liponsäure und Silibinin einem engen Konzentrationsbereich um 70 µg/ml eine vollständige Restitution des Thiolstatus der Alveolarmakrophagen initiierte. Wie in Tabelle 3 dargestellt, wurden signifikante Steigerungen des Thiolgehaltes über den gesamten Untersuchungszeitraum für diese Silibinindosis nachgewiesen. Die alleinige Wirkung der α-Liponsäure wurde regelmäßig signifikant überschritten. Geringere additive Konzentrationen von Silibinin induzierten nach einer längeren Behandlungsdauer ebenfalls eine Thiolrestitution.

Die Beeinflussung membranständiger Thiole war, wie in Tabelle 4 belegt, in den oben genannten Konzentrations-Zeitregimen nachweisbar. Im Gegensatz zu der intrazellulären Induktion wurden Oberflächenthiole bereits nach 24 Stunden in Gegenwart geringerer Silibininkonzentrationen moduliert.

Tabelle 3 zeigt den Einfluss von α-Liponsäure in Kombination mit Silibinin auf die intrazelluläre Thiolkonzentration einer Alveolarmakrophagen-Zelllinie [*: p<0,05, ANOVA, n=12] .

**Tabelle 3:**

| Thiole, intrazellulär | 24h | 48h | 72h | 96h |
|---|---|---|---|---|
| (CMFDA) [%] | MW ± SD | MW ± SD | MW ± SD | MW ± SD |
| KO, normal RPMI1640 | 100,0 ± 29,0 | 100,0 ± 29,3 | 100,0 ± 52,4 | 100,0 ± 14,2 |
| KO, thioldefizient TDM | 77,4 ± 26,6 | 86,8 ± 4,0 | 88,8 ± 50,0 | 82,1 ± 13,7 |
| αLS [10,0 µg/ml] TDM | 69,2 ± 28,2 | 91,1 ± 16,6 | 88,6 ± 26,2 | 114,8 ± 33,2 |
| αLS[10,0 µg/ml] Silibinin [0,07 µg/ml] TDM | 77,5 ± 26.8* | 91,5 ± 17,3 | 92,9 ± 45,0 | 122,6 ± 33,1* |
| αLS[10,0 µg/ml] Silibinin [0,7 µg/ml] TDM | 75,1 ± 27,4 | 93,1 ± 24,7 | 90,0 ± 37,8 | 108,2 ± 22,9* |
| αLS[10,0 µg/ml] Silibinin [7,0 µg/ml] TDM | 73,3 ± 2,3 | 90,4 ± 29,0 | 85,9 ± 35,7 | 124,2 ± 40,1* |
| αLS [10,0 µg/ml] Silibinin [70,0 µg/ml] TDM | 161,7 ± 76,7* | 173,4 ± 85,2* | 126,6 ± 29,5* | 143,3 ± 51,9* |
| αLS[10,0 µg/ml] Silibinin [700,0 µg/ml] TDM | 40,9 ± 17,4 | 18,7 ± 10,5 | 17,1 ± 11,6 | 22,5 ± 7,5 |

Tabelle 4 zeigt den Einfluss von α-Liponsäure in Kombination mit Silibinin auf die membranständige Thiolkonzentration einer Alveolarmakrophagen-Zelllinie [*: p<0,05, ANOVA, n=12] .

**Tabelle 4:**

| Thiole, membranständig | 24h | 48h | 72h | 96h |
|---|---|---|---|---|
| (CMFDA) [%] | MW ± SD | MW ± SD | MW ± SD | MW ± SD |
| KO, normal RPMI1640 | 100,0 ± 6,8 | 100,0 ± 8,2 | 100,0 ± 34,5 | 100,0 ± 47,0 |
| KO, thioldefizient TDM | 82,5 ± 3.6 | 88,9 ±7,3 | 87,5 ± 35,7 | 100,2 ±54,5 |
| αLS[10,0 µg/ml] TDM | 108,8 ± 38,2 | 126,2 ± 68,6 | 90,7 ± 28,1 | 107,3 ± 57,4 |
| αLS[10,0 µg/ml] Silibinin [0,07 µg/ml] TDM | 103,4 ± 54,6 | 126,7 ± 56,8 | 106,3 ± 39,5 | 109,9 ± 60,9 |
| αLS[10,0 µg/ml] Silibinin [0,7 µg/ml] TDM | 124,3 ± 21,7* | 133,8 ± 54,2* | 91,8 ± 36,6 | 110,9 ± 45,9 |
| αLS[10,0 µg/ml] Silibinin [7,0 µg/ml] TDM | 109,4 ± 32,9* | 175,2 ± 65,8* | 135,7 ± 21,0* | 111,2 ± 30,4 |
| αLS[10,0 µg/ml] Silibinin [70,0 µg/ml] TDM | 150,0 ± 24,1* | 138,7 ± 62,4* | 102,0 ± 45,0* | 110,5 ± 44,2 |
| αLS[10,0 µg/ml] Silibinin [700,0 µg/ml] TDM | 68,3 ± 4,0 | 103,1 ± 26,1 | 91,1 ± 32,9 | 122,1 ± 39,2 |

### vergleichsbeispiel 3

### Einfluss der Kombination von α-Liponsäure in Kombination mit Ambroxol auf den zellulären Thiolstatus von primären Alveolarmakrophagen von COPD Patienten

Alveolarmakrophagen wurden aus der bronchoalveolären Lavageflüssigkeit von COPD Patienten isoliert, in Zellkulturmedium aufgenommen und in einem Begasungsbrutschrank bei 37°C, einer relativen Luftfeuchte von 98% und 7,5% relativem Luft-CO₂-Gehalt inkubiert. Um den Einfluss der erfindungsgemäß zur Anwendung kommenden Kombinationen auf den Thiolstatus der Peritonealmakrophagen zu prüfen, wurden jeweils eine Fraktion mit α-Liponsäure, dem Effektor des Glutathionmetabolismus Ambroxol bzw. mit der Kombination von α-Liponsäure / Ambroxol behandelt, während jeweils eine weitere Fraktion als unbehandelte Kontrolle geführt wurde. Unbehandelte Alveolarmakrophagen von gesunden, nicht-COPD Patienten dienten als Normalvergleich

Die Bestimmung des zellulären Thiolstatus erfolge mittels der unter 1. beschriebenen Meßmethode. In der Tabelle 5 ist der Effekt der Kombination von α-Liponsäure und Ambroxol in der Zeitkinetik über 96 Stunden in Relation zu Gesunden dargestellt.

Unter Zusatz der Monosubstanzen war nur ein marginaler Anstieg der zellulären Thiolexpression unter Verwendung von Ambroxol zu beobachten, während α-Liponsäure keinen Effekt zeigte. Demgegenüber war unter der Kombination von α-Liponsäure und Ambroxol beginnend nach 24 Stunden ein deutlicher Anstieg der zellulären Thiolexpression nachweisbar, der in Gegenwart von 10 µM Ambroxol ein superadditives und signifikantes Maximum im gesamten Untersuchungszeitraum erreichte.

Tabelle 5 zeigt den Einfluss von α-Liponsäure in Kombination mit Ambroxol auf die zelluläre Thiolkonzentration primärer Alveolarmakrophagen von COPD-Patienten [*: p<0,05, ANOVA, n=8].

**Tabelle 5:**

| Thiole, intrazellulär | 24h | 48h | 72h | 96h |
|---|---|---|---|---|
| (CMFDA) [%] | MW ± SD | MM ± SD | MW ± SD | MW ± SD |
| Kontrolle normal RPMI1640 | 100,0 ± 19.7 | 100,0 ± 13,1 | 100,0 ± 31,8 | 100,0 ± 23,2 |
| Kontrolle, COPD, RPMI1640 | 61,6 ± 13,9 | 58,6 ± 13,7 | 46,1 ± 18,8 | 51,9 ± 14,3 |
| αLS [10,0 µg/ml] RPMI1640 | 60,3 ± 21,0 | 58,0 ± 17,0 | 46,2 ± 19,8 | 62,4 ± 6,3 |
| Ambroxol [10,0 µ/ml] RPMI1640 | 67,4 ± 16,4 | 61,8 ± 11,5 | 61,6 ± 20,8 | 67,1 ± 8,4 |
| αLS[10,0 µg/ml] Ambroxol [1,0 µM] RPMI1640 | 81,1 ± 15,7* | 81,9 ± 10,1* | 83,9 ± 18,2* | 81,2 ± 8,8* |
| αLS [10,0 µg/ml] Ambroxol [10,0 µM] RPMI1640 | 101,2 ± 17,7* | 109,5 ± 13,4* | 113,9 ± 25,6* | 107,8 ± 26,8* |
| αLS [10,0 µg/ml] Ambroxol [100,0 µM] RPMI1640 | 84,7 ± 25,5 | 79,6 ± 13,4 | 70,0 ± 31,8* | 76,6 ± 9,7 |
| αLS [10,0 µg/ml] Ambroxol (1000,0 µM) RPMI1640 | 31,5 ± 12,1 | 53,8 ± 22,8 | 34,8 ± 2,1 | 36,1 ± 4,2 |

### Beispiel 4

### Einfluss der Kombination von α-Liponsäure in Kombination mit Silibinin auf den zellulären Thiolstatus von primären Alveolarmakrophagen von COPD Patienten

In einer, dem Beispiel 3 identischen Versuchsanordnung wurden Alveolarmakrophagen von COPD Patienten jeweils mit α-Liponsäure, dem Effektor Silibinin bzw. mit der Kombination von α-Liponsäure / Silibinin behandelt, während wiederum jeweils eine weitere Fraktion als unbehandelte Kontrolle geführt wurde. Unbehandelte Alveolarmakrophagen von gesunden, nicht-COPD Patienten dienten auch hier als Normalvergleich.

Die Bestimmung des zellulären Thiolstatus erfolgte mittels der unter 1. beschriebenen Meßmethode. In der Tabelle 6 ist der Effekt der Kombination von α-Liponsäure und Silibinin in der Zeitkinetik über 96 Stunden in Relation zu Gesunden dargestellt.

Unter Zusatz der Monosubstanzen α-Liponsäure oder Silibinin war keine Modulation der zellulären Thiolexpression zu beobachten. Demgegenüber war unter der Kombination von α-Liponsäure und Silibinin beginnend nach 24 Stunden ein deutlicher Anstieg der zellulären Thiolexpression nachweisbar, der in Gegenwart von 70 µg/ml Silibinin ein superadditives und signifikantes Maximum im gesamten Untersuchungszeitraum erreichte.

Tabelle 6 zeigt den Einfluss von α-Liponsäure in Kombination mit Silibinin auf die zelluläre Thiolkonzentration primärer Alveolarmakrophagen von COPD-Patienten [*: p<0,05, ANOVA, n=8].

**Tabelle 6:**

| Thiole, intrazellulär | 24h | 48h | 72h | 96h |
|---|---|---|---|---|
| (CMFDA) [%] | MW ± SD | MW ± SD | MW ± SD | MW ± SD |
| Kontrolle, normal RPMI1640 | 100,0 ± 19,7 | 100,0 ± 8,2 | 100,0 ± 31,8 | 100,0 ± 23,2 |
| Kontrolle, COPD, RPMI1640 | 61,6 ± 13,9 | 58,6 ± 7,3 | 46,1 ± 18,8 | 51,9 ± 14,3 |
| αLS [10,0 µg/ml] RPMI1640 | 60,3 ± 21,0 | 58,0 ± 68,6 | 46,2 ± 19,8 | 62,4 ± 6,3 |
| Silibinin [70 µg/ml] RPMI1640 | 56,1 ± 12,4 | 59,0 ± 56,8 | 44,7 ± 14,0 | 49,4 ± 14,5 |
| αLS[10,0 µg/ml] Silibinin [0,7 µg/ml] RPMI1640 | 64,1 ± 10,2* | 64,7 ± 54,2* | 49,9 ± 11,8 | 62,6 ± 8,0 |
| αLS[10,0 µg/ml] Silibinin [7,0 µg/ml] RPMI1640 | 84,5 ± 14,1* | 76,0 ± 65,8* | 78,6 ± 14,9* | 81,8 ± 17,8 |
| αLS[10,0 µg/ml] Silibinin [70,0 µg/ml] RPMI1640 | 102,5 ± 22,6* | 103,3 ± 62,4* | 100,0 ± 27,1* | 92,7 ± 20,1* |
| αLs[10,0 µg/ml] Silibinin [700,0 µg/ml] RPMI1640 | 59,4 ± 11, 7 | 36,2 ± 26,1 | 38,8 ± 10,3 | 35,7 ± 2,7 |

### Beispiel 5

### Einfluss auf die Phagozytosefähigkeit von Alveolarmakrophagen

Um eine Charakterisierung der Alveolarmakrophagen hinsichtlich ihrer originären Funktionen zu ermöglichen, wurde die Phagozytosefähigkeit als Messgröße ausgewählt.

Alveolarmakrophagen wurden analog des im Beispiel 3 beschriebenen Vorgehens isoliert und ex vivo kultiviert. Die Bestimmung der Phagozytoseleistung erfolgte durch einen zytofluorimetrischen Test auf Einzelzellebene. Dabei wurden die Makrophagen mit opsonierten und fluorochrommarkierten Bakterien cokultiviert. Die Menge der in einem definierten Zeitraum aufgenommenen Bakterien wurde quantitativ über die Fluoreszenzintensität in den Makrophagen erfasst und galt als Maß für deren Phagozytosekapazität. Der Einfluss der erfindungsgemäß zur Anwendung kommenden Kombinationen auf die Phagozytosefähigkeit der Peritonealmakrophagen nach einer Behandlungsdauer von bis zu 96 Stunden ist in der Tabelle 7 dargestellt.

Nach Inkubation mit α-Liponsäure und Silibinin bzw. Ambroxol als Referenz war die Phagozytoserate gegenüber der unbehandelten Kontrolle nicht verändert. Demgegenüber konnte unter Verwendung der Kombination von α-Liponsäure und Ambroxol eine signifikante Steigerung der Phagozytoserate erreicht werden, die nach 72 Stunden den Werten der gesunden Kontrollgruppe entsprach.

Ähnlich war der Verlauf der Phagozytoseinduktion unter der Kombination von α-Liponsäure und Silibinin in einer Konzentration von 70 µg/ml. Auch hier wurde eine signifikante Verbesserung der Phagozytosekapazität parallel zu einer Restitution des Thiolstatus nachgewiesen.

Tabelle 7 zeigt den Einfluss von α-Liponsäure in Kombination mit Ambroxol als Referenz bzw. Silibinin auf die Phagozytoserate primärer Alveolarmakrophagen von COPD-Patienten [*: p<0,05, ANOVA, n=6].

**Tabelle 7:**

| Phagoburst | 24h | 48h | 72h | 96h |
|---|---|---|---|---|
| (mfi) [%] | MW ± SD | MW ± SD | MW ± SD | MW ± SD |
| Kontrolle, normal RPMI1640 | 100,0 ± 16,8 | 100,0 ± 22,2 | 100,0 ± 14,6 | 100,0 ± 13.7 |
| Kontrolle, COPD, RPMI1640 | 56,9 ± 15,5 | 55,4 ± 14,1 | 57,0 ± 9,7 | 67,7 ± 13,7 |
| αLS [10,0 µg/ml] RPMI1640 | 59,0 ± 11,7 | 60,2 ± 12,4 | 50,7 ± 9,0 | 64,9 ± 14,3 |
| Referenz Ambroxol [10 µM] RPMI1640 | 53,3 ± 10,9 | 59,2 ± 12,4 | 55,2 ± 19,2 | 61,9 ± 22,1 |
| Silibinin [70,0 µg/ml] RPMI1640 | 55,1 ± 12,2* | 57,6 ± 8,5 | 54,2 ± 13,8 | 59,9 ± 21,2 |
| αLS [10,0 µg/ml] Referenz Ambroxol [10 µM] RPMI1640 | 81,5 ± 19,0* | 93,2 ± 18,4* | 116,4 ± 17,6* | 102,8 ± 4,8* |
| αLS [10,0 µg/ml] Silibinin [70,0 µg/ml] RPMI1640 | 75,5 ± 17,3* | 86,5 ± 16,7* | 98,7 ± 22, 6* | 92,5 ± 9,1* |

Insgesamt machen diese Versuche deutlich, dass die Applikation der Kombination von α-Liponsäure und den Effektoren des Glutathionmetabolismus Ambroxol bzw. Silibinin einen primär massiv geschädigten Thiolstatus in thioldefizienten Alveolarmakrophagen sowohl nach artifizieller Thioldefizienz als auch bei COPD-Patienten stabilisiert. Durch diese Normalisierung kommt es darüberhinaus zu einer Wiederherstellung zentraler zellulärer Funktionen, wie der Phagozytoseaktivität, welche ohne eine solche Behandlung nicht zu verzeichnen ist.

## Patentansprüche

1. Verwendung von Silibinin und/oder dessen Salze zusammen mit α-Liponsäure und/oder deren Salze für die Herstellung eines Arzneimittels zur gleichzeitigen, getrennten oder zeitlich abgestuften zytoprotektiven Behandlung chronisch obstruktiver Lungenerkrankungen.

2. Verwendung nach Anspruch 1,
**dadurch gekennzeichnet, dass** die Dosis der α-Liponsäure und/oder deren Salze für die humanmedizinische Applikation beim Patienten zwischen 30 und 1800 mg/d, bevorzugt zwischen 200 und 600 mg/d liegt.

3. Verwendung nach einem der Ansprüche 1 oder 2,
**dadurch gekennzeichnet, dass** die Dosis von Silibinin und/oder dessen Salze für die humanmedizinische Applikation beim Patienten zwischen 20 und 1600 mg/d, bevorzugt zwischen 300 und 800 mg/d liegt.

4. Verwendung nach mindestens einem der vorhergehenden Ansprüche,
**dadurch gekennzeichnet, dass** das Arzneimittel inhalativ, oral oder parenteral verabreichbar ist.

5. Verwendung nach mindestens einem der vorhergehenden Ansprüche,
**dadurch gekennzeichnet, dass** das Arzneimittel weitere Additive ausgewählt aus der Gruppe wässriger Lösungsmittel, Stabilisatoren, Suspensions-, Dispersions- und Benetzungsmittel enthält.

6. Verwendung nach mindestens einem der vorhergehenden Ansprüche,
**dadurch gekennzeichnet, dass** das Arzneimittel in Form eines Aerosols, einer Lösung, eines Granulats, eines Pulver, einer Emulsion, einer Tablette und/oder einer Filmtablette vorliegt.

7. Verwendung nach mindestens einem der vorhergehenden Ansprüche,
**dadurch gekennzeichnet, dass** Silibinin und/oder dessen Salze und die α-Liponsäure und/oder deren Salze in einer einzigen Formulierung vorliegen.

8. Verwendung nach mindestens einem der vorhergehenden Ansprüche,
**dadurch gekennzeichnet, dass** Silibinin und/oder dessen Salze und die α-Liponsäure und/oder deren Salze in getrennten Formulierungen vorliegen.

## Claims

1. Use of silibinin and/or its salts with α-lipoic acid and/or its salts for the manufacture of a medicament for the simultaneous, separate or timed cytoprotective treatment of chronically obstructive lung diseases.

2. Use according to claim 1,
**characterised in that** the dose of the α-lipoic acid and/or its salts for administration to a human patient is between 30 and 1800 mg/d, preferably between 200 and 600 mg/d.

3. Use according to one of claims 1 or 2,
**characterised in that** the dose of silibinin and/or its salts for administration to a human patient is between 20 and 1600 mg/d, preferably between 300 and 800 mg/d.

4. Use according to at least one of the preceding claims,
**characterised in that** the medicament can be administered by inhalation, orally or parenterally.

5. Use according to at least one of the preceding claims,
**characterised in that** the medicament contains further additives selected from the group of aqueous solvents, stabilisers, suspending, dispersing and wetting agents.

6. Use according to at least one of the preceding claims,
**characterised in that** the medicament is presented in the form of an aerosol, a solution, granules, a powder, an emulsion, a tablet and/or a film tablet.

7. Use according to at least one of the preceding claims,
**characterised in that** silibinin and/or its salts and the α-lipoic acid and/or its salts are presented in a single formulation.

8. Use according to at least one of the preceding claims,
**characterised in that** silibinin and/or its salts and the α-lipoic acid and/or its salts are presented in separate formulations.

## Revendications

1. Utilisation de silibinine et/ou de ses sels conjointement avec de l'acide α-lipoïque et/ou ses sels, pour la fabrication d'un médicament destiné au traitement cytoprotecteur simultané, séparé ou échelonné dans le temps de broncho-pneumopathies chroniques obstructives.

2. Utilisation selon la revendication 1, **caractérisée en ce que** la dose de l'acide α-lipoïque et/ou de ses sels pour l'administration au patient en médecine humaine est comprise entre 30 et 1 800 mg par jour, de préférence entre 200 et 600 mg par jour.

3. Utilisation selon la revendication 1 ou 2, **caractérisée en ce que** la dose de silibinine et/ou de ses sels pour l'administration au patient en médecine humaine est comprise entre 20 et 1 600 mg par jour, de préférence entre 300 et 800 mg par jour.

4. Utilisation selon au moins l'une des revendications précédentes, **caractérisée en ce que** le médicament peut être administré par voie orale ou parentérale ou par inhalation.

5. Utilisation selon au moins l'une des revendications précédentes, **caractérisée en ce que** le médicament contient d'autres additifs choisis dans le groupe constitué par des solvants, des stabilisants, des agents de mise en suspension, des dispersants et des agents mouillants.

6. Utilisation selon au moins l'une des revendications précédentes, **caractérisée en ce que** le médicament se trouve sous forme d'un aérosol, d'une solution, d'un produit granulé, d'une poudre, d'une émulsion, d'un comprimé et/ou d'un comprimé pelliculé.

7. Utilisation selon au moins l'une des revendications précédentes, **caractérisée en ce que** la silibinine et/ou ses sels et l'acide α-lipoïque et/ou ses sels sont présents dans une forme galénique unique.

8. Utilisation selon au moins l'une des revendications précédentes, **caractérisée en ce que** la silibinine et/ou ses sels et l'acide α-lipoïque et/ou ses sels sont présents dans des formes galéniques distinctes.
